# EUROPEAN PATENT SPECIFICATION

(11) **EP 1 575 555 B1**
(45) Date of publication and mention of the grant of the patent: **06.08.2014**
(21) Application number: 03811862.6
(22) Date of filing: 21.11.2003
(51) Int. Cl.: A61K 9/00

(54) **FORMULATIONS OF FINASTERIDE**
ZUBEREITUNGEN VON FINASTERIDEN
FORMULATIONS DE FINASTERIDE

(30) Priority: 22.11.2002 IS 663302
(43) Date of publication of application: 21.09.2005
(73) Proprietor: Actavis Group hf., 220 Hafnarfjordur (IS)
(72) Inventor: JOHANNSSON, Fjalar, IS-103 Reykjavik (IS); ARNASON, Birkir, IS-107 Reykjavik (IS)
(74) Representative: Arnason Faktor
(86) International application number: PCT/IS2003/000034
(87) International publication number: WO 2004/047798

(56) References cited:
- WO-A-01/37808
- WO-A-99/08666
- WO-A-99/08684
- WO-A-99/21534
- US-A- 5 433 951
- US-B1- 6 294 192

## Description

### FIELD OF THE INVENTION

The present invention relates to improved dissolution of finasteride tablet formulations.

### TECHNICAL BACKGROUND AND PRIOR ART

Finasteride, 17β-(N-tert-butyl carbamoyl)-4-aza-5α-androst-1-en-3-one is a 5α-reductase inhibitor for use in treating acne, female hirsutism, male pattern hair loss (androgenic alopecia) and particularly benign prostatic hyperplasia. See for example U.S. patent No. 4,377,584 and Wilde and Goa, Finasteride, an update of its use in the management of symptomatic benign prostatic hyperplasia, Drugs, Vol. 57, No. 4, April 1999, pp 557-581.

The preparation of useful formulations of finasteride is complicated since finasteride is practically insoluble in water. Hydrophobic therapeutic agents like finasteride present difficult problems in formulating such compounds for effective administration to patients. A well-designed formulation must, at a minimum, be capable of presenting a therapeutically effective amount of the hydrophobic compound to the desired absorption site, in an absorbable form.

WO 99/08666 and WO 99/08684 relate to novel solutions of aza steroid (finasteride included) in combination with fatty acid ester of glycerol or propylene glycol.

US 6,294,192 relates to triglyceride-free pharmaceutical compositions for delivery of hydrophobic therapeutic agents.

In an attempt to improve the solubility of the active ingredient numerous wetting agents were tried. It has now been discovered that useful formulations of finasteride can be produced by use of Gelucire®.

Gelucire® is used in various applications including preparing sustained release pharmaceutical compositions, as described in the technical and patent literature. For example, U.S. Pat. No. 5,433,951 describes sustained release formulation containing captopril as the active ingredient.

U.S. Pat. No. 6,171,615 describes a stable sustained release theophylline formulation which is prepared by incorpurating theophylline into a semisolid matrix comprising Gelucire.

U.S. Pat. No. 6,312,704 describes compositions providing enhanced bioavailability by mixing together a lipophilic phase, a surfactant, a cosurfactant an a pharmaceutical active ingredient. Gelucire 44/14 is one of the polyglycolized glycerides mentioned.

Sheen et al, Bioavailability of a Poorly Water-Soluble Drug from Tablet and Solid Dispersions in Humans, J. Pharm. Sci., Vol. 80, No. 7, July 1991, pp 712 to 714, discloses the use of Gelucire 44/14-polyethylene glycol (PEG) 400 mixture as carrier for REV 5901 (α-pentyl-3-(2-quinolinylmethoxy)benzenemethanol, a 5-lipoxygenase inhibitor) in a tablet. REV 5901 is a water-insoluble drug and the Gelucire 44/14-PEG 400 mixture provided improved dissolution thereof to effect faster release.

A. Ainaoiu, E.M. Ouriemchi, et al, Process of Drug Release with Oral Dosage Forms with a Lipidic Gelucire Matrix, Journal of Polymer Engineering, Vol. 17, No. 3, 1997, pp 245 to 255, discloses a study of drug release out of dosage forms made of the drug dispersed in Gelucire. The study concerns controlled release of the drug sodium salicylate.

D. Bidah, E.M Ouriemchi, et al, Diffusion Process of Drug Delivery from a Dosage Form wiht a Gelucire Matrix, No. 80, 1992, pp 145-149, describes a study of dosage forms having the property of delivering the drug at a controlled rate, with the drug, sodium salicylate, being dispersed in Gelucire, playing the role of a matrix, in syntetic gastric liquid.

None of the above references mention the use of Gelucire with finasteride.

### DETAILED DESCRIPTION

The invention provides a pharmaceutical tablet formulation comprising finasteride, 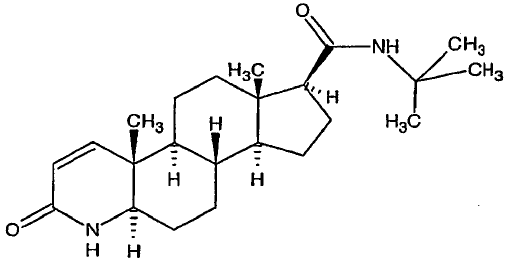 a wetting agent and a binding agent as defined in the claims.

The pharmaceutical formulation of the present invention comprises 0.1-10 wt% of finasteride, 0-10 wt% of the wetting agent and 0-90 wt% of microcristalline cellulose.

The wetting agent may suitably be selected from Gelucire®, docusate sodium, sodium lauryl sulfate and polysorbate. Gelucire® is particularly preferred.

Gelucire® is a well-defined mixture of mono-, di- and triglycerides and mono- and di-fatty acid esters of polyethylene glycol, wherein the predominant fatty acid is lauric acid.

The binding agent may suitably also be selected from gelatin, dextrin, povidone or starch but microcrystalline cellulose is particularly preferred.

The formulation may additionally comprise a further pharmaceutically active compound, such as epristeride and zanosterone; filler material such as cellulose and starch; and lubricant such as magnesium stearate.

These formulations may also comprise a finasteride analogs instead of finasteride.

### EXAMPLES

### Example 1

The following materials were combined by wet granulation to produce 5 mg finasteride tablets:

| | |
|---|---|
| Finasteride | 5.0 mg |
| Lactose monohydrate powder | 103.3 mg |
| Cellulose microcrystalline | 15.0 mg |
| Starch maiza pregel. | 15.0 mg |
| Gelucire 44/14® | 3.0 mg |
| Sodium starch glyc. | 7.5 mg |
| Talcum | 0.4 mg |
| Magnesium stearate | 0.8 mg |

### Example 2

The following materials were combined by wet granulation to produce 5 mg finasteride tablets:

| | |
|---|---|
| Finasteride | 5.0 mg |
| Lactose monohydrate powder | 88.7 mg |
| Cellulose microcrystalline, | 30.0 mg |
| Starch maiza pregel. | 15.0 mg |
| Gelucire 44/14® | 3.0 mg |
| Sodium starch glyc. | 7.5 mg |
| Magnesium stearate | 0.8 mg |

### Example 3

The following materials were combined by wet granulation to produce 5 mg finasteride tablets:

| | |
|---|---|
| Finasteride | 5.0 mg |
| Lactose monohydrate powder | 104.4 mg |
| Cellulose microcrystalline | 15.0 mg |
| Starch maiza pregel. | 15.0 mg |
| Gelucire 44/14® | 2.3 mg |
| Sodium starch glyc. | 7.5 mg |
| Magnesium stearate | 0.8 mg |

### Example 4

The following materials were combined by wet granulation to produce 5 mg finasteride tablets:

| | |
|---|---|
| Finasteride | 5.0 mg |
| Lactose monohydrate powder | 79.7 mg |
| Cellulose microcrystalline | 37.5 mg |
| Starch maiza pregel. | 15.0 mg |
| Gelucire 44/14® | 4.5 mg |
| Sodium starch glyc. | 7.5 mg |
| Magnesium stearate | 0.8 mg |

### Example 5

The following materials were combined by wet granulation to produce 5 mg finasteride tablets:

| | |
|---|---|
| Finasteride | 5.0 mg |
| Lactose monohydrate powder | 105.6 mg |
| Cellulose microcrystalline, | 15.0 mg |
| Starch maiza pregel. | 15.0 mg |
| Gelucire 44/14® | 1.1 mg |
| Sodium starch glyc. | 7.5 mg |
| Magnesium stearate | 0.8 mg |

### Example 6

The following materials were combined by wet granulation to produce 5 mg finasteride tablets:

| | |
|---|---|
| Finasteride | 5.0 mg |
| Lactose monohydrate powder | 105.2 mg |
| Cellulose microcrystalline | 15.0 mg |
| Starch maiza pregel., | 15.0 mg |
| Gelucire 44/14® | 1.5 mg |
| Sodium starch glyc. | 7.5 mg |
| Magnesium stearate | 0.8 mg |

### Example 7

The following materials were combined by wet granulation to produce 5 mg finasteride tablets:

| | |
|---|---|
| Finasteride | 5.0 mg |
| Lactose monohydrate powder | 106.3 mg |
| Cellulose microcrystalline | 15.0 mg |
| Starch maiza pregel. | 15.0 mg |
| Gelucire 44/14® | 0.4 mg |
| Sodium starch glyc. | 7.5 mg |
| Magnesium stearate | 0.8 mg |

### Example 8

Disintegration time and friability of finasteride formulations

| | Disentegrationa time [min:sec] | Friability [%] |
|---|---|---|
| Example 1 | 1:40 | 0.93 |
| Example 2 | 3:00 | 0.30 |
| Example 3 | 2:30 | 0.60 |
| Example 4 | 2:40 | 0.46 |
| Example 5 | 1:45 | 0.66 |
| Example 6 | 1:00 | 0.40 |
| Example 7 | 1:00 | 0.33 |

### Example 9

Comparision of dissolution speed between formulations including the wetting agents Gelucire®, polysorbate 80, sodium lauryl sulfate and docusate sodium.

| | FI111-15 | FI111-4 | FI111-7 | FI111-8 |
|---|---|---|---|---|
| Finasteride | 5.0 | 5.0 | 5.0 | 5.0 |
| Lactose monohydrate powder | 105.2 | 106.1 | 103.3 | 105.9 |
| Cellulose microcrystalline | 15.0 | 15.0 | 15.0 | 15.0 |
| Starch maiza pregel. | 15.0 | 15.0 | 15.0 | 15.0 |
| Gelucire 44/14® | 1.5 | | | |
| Polysorbate 80 | | 0.4 | | |
| Sodium lauryl sulfate | | | 3.0 | |
| Docusate sodium | | | | 0.4 |
| Sodium starch glyc. | 7.5 | 7.5 | 7.5 | 7.5 |
| Talcum | | | 0.4 | 0.4 |
| Magnesium stearate | 0.8 | 0.8 | 0.8 | 0.8 |

**Table 1: % dissolved finasteride with different wetting agents**

| | **FI111-4** | **FI111-7** | **FI111-8** | **FI111-15** |
|---|---|---|---|---|
| time [min] | Polysorbate | Sodium lauryl sulfate | Docusate sodium | Gelucire |
| 0.0 | 0,0 | 0,0 | 0,0 | 0,0 |
| 5,0 | 29,5 | 28,3 | 45,8 | 68,2 |
| 7,0 | 43,2 | 45,3 | 62,4 | 80,3 |
| 10,0 | 50,3 | 54,7 | 71,8 | 85,8 |
| 15,0 | 56,9 | 83,6 | 79,5 | 89,1 |
| 20,0 | 61,0 | 69,8 | 83,4 | 92,1 |
| 30,0 | 67,5 | 74,8 | 87,2 | 92,1 |
| 45,0 | 73,4 | 80,4 | 89,5 | 93,1 |
| 60,0 | 76,0 | 83,7 | 90,9 | 92,6 |

Examples 2-7 show various concentrations of Gelucire® and microcrystalline cellulose.

Example 8 shows results in disintegration time and friability for the formulations in Examples 2-7.

Example 9 shows different dissolution profiles for different wetting agents in the formulation.

By using Gelucire 44/14® the dissolution profile of the finasteride improved to a satisfactory level. However the binding of the tablets decreased as the quantity of Gelucire increased. In example 1 (2% Gelucire concentration) the friability of the tablets became almost 1%. In an achivement to improve the binding, the levels of microcrystalline cellulose were increased.

### Description of Figures

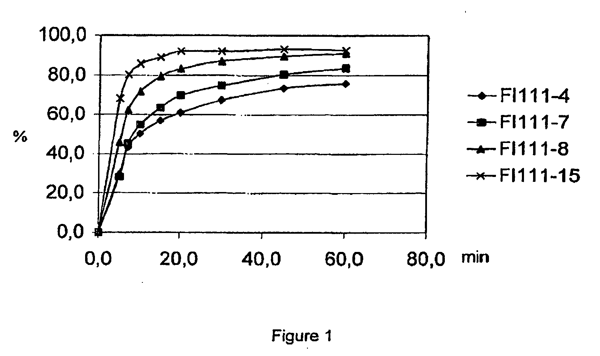

Figure 1 shows dissolution speed for finasteride formulations, showing % dissolved finasteride vs. time (min).

## Claims

1. A pharmaceutical tablet formulation comprising:
a. finasteride;
b. a wetting agent selected from Gelucire®, sodium lauryl sulfate and polysorbate,
c. a binding agent selected from gelatin, dextrin, povidone, starch and microcrystalline cellulose,
optionally in combination with other excipients.

2. The tablet formulation of claim 1 wherein the binding agent comprises microcrystalline cellulose.

3. The tablet fomulation of claim 1, wherein the wt/wt ratio of the wetting agent and the binding agent is in the range of 0.01-1.0.

4. The tablet formulation of claim 1, wherein the wetting agent is Gelucire®.

5. The tablet formulation of claim 3, wherein the Gelucire® is Gelucire® 44/14.

6. The tablet formulation of claim 1, which comprises finasteride, Gelucire®, microcrystalline cellulose, lactose monohydrate powder, starch, sodium starch glycolate and magnesium stearate.

## Patentansprüche

1. Pharmazeutische Tablettenformulierung, umfassend:
a. Finasterid,
b. ein Netzmittel ausgewählt aus Gelucire®, Natriumlaurylsulfat und Polysorbat,
c. ein Bindemittel ausgewählt aus Gelatine, Dextrin, Povidon, Stärke und mikrokristalliner Cellulose,
gegebenenfalls in Kombination mit anderen Exzipienten.

2. Tablettenformulierung nach Anspruch 1, wobei das Bindemittel mikrokristalline Cellulose umfasst.

3. Tablettenformulierung nach Anspruch 1, wobei das Gewichtsverhältnis von Netzmittel zu Bindemittel im Bereich von 0,01-1,0 liegt.

4. Tablettenformulierung nach Anspruch 1, wobei es sich bei dem Netzmittel um Gelucire® handelt.

5. Tablettenformulierung nach Anspruch 3, wobei es sich bei Gelucire® um Gelucire® 44/14 handelt.

6. Tablettenformulierung nach Anspruch 1, welche Finasterid, Gelucire®, mikrokristalline Cellulose, Lactosemonohydratpulver, Stärke, Natriumstärkeglykolat und Magnesiumstearat umfasst.

## Revendications

1. Formulation de comprimé pharmaceutique, comprenant :
a. du finastéride ;
b. un agent mouillant choisi parmi le Gelucire®, le laurylsulfate de sodium et un polysorbate ;
c. un liant choisi parmi la gélatine, une dextrine, la povidone, l'amidon et la cellulose microcristalline ;
éventuellement en combinaison avec d'autres excipients.

2. Formulation de comprimé selon la revendication 1, dans laquelle le liant comprend de la cellulose microcristalline.

3. Formulation de comprimé selon la revendication 1, dans laquelle le rapport pondéral de l'agent mouillant au liant se trouve dans la plage de 0,01-1,0.

4. Formulation de comprimé selon la revendication 1, dans laquelle l'agent mouillant est le Gelucire®.

5. Formulation de comprimé selon la revendication 3, dans laquelle le Gelucire® est le Gelucire® 44/14.

6. Formulation de comprimé selon la revendication 1, qui comprend du finastéride, du Gelucire®, de la cellulose microcristalline, de la poudre de lactose monohydraté, de l'amidon, du glycolate d'amidon sodique et du stéarate de magnésium.
